# EUROPEAN PATENT SPECIFICATION

(11) **EP 1 279 038 B1**
(45) Date of publication and mention of the grant of the patent: **24.11.2004**
(21) Application number: 01973779.0
(22) Date of filing: 30.04.2001
(51) Int. Cl.: G01N 33/50

(54) **METHOD OF IDENTIFYING AGENTS THAT ALTER THE ACTIVITY OF THE PROMOTER SEQUENCE FOR CRF RECEPTOR 2-ALPHA**
VERFAHREN ZUM NACHWEIS VON AGENTIEN DIE DIE AKTIVITÄT DER PROMOTERSEQUENZ DES CRF-2 ALPHA REZEPTORS VERÄNDERN
PROCEDE POUR IDENTIFIER DES AGENTS QUI AFFECTENT L'ACTIVITE DE LA SEQUENCE PROMOTRICE DU RECEPTEUR DE CORTICOLIBERINE CRF 2-ALPHA

(30) Priority: 02.05.2000 US 201129 P
(43) Date of publication of application: 29.01.2003
(73) Proprietor: WISCONSIN ALUMNI RESEARCH FOUNDATION, Madison, WI 53705 (US)
(72) Inventor: KALIN, Ned, H., Madison, WI 53705 (US); ROSEBOOM, Patrick, H., Madison, WI 53711 (US); LANDRY, Charles, F., Fitchburg, WI 53711 (US); NANDA, Steven, A., Madison, WI 53719 (US)
(74) Representative: Duckworth, Timothy John
(86) International application number: PCT/US2001/013920
(87) International publication number: WO 2001/083563

(56) References cited:
- US-A- 5 965 790
- ECKART ET AL: "Actions of CRF and its analogs" CURRENT MEDICINAL CHEMISTRY, BENTHAM SCIENCE PUBLISHERS BV, BE, vol. 6, no. 11, November 1999 (1999-11), pages 1035-1053, XP000882144 ISSN: 0929-8673
- SPIESS ET AL: "Molecular properties of the CRF receptor" TRENDS IN ENDOCRINOLOGY AND METABOLISM, ELSEVIER SCIENCE PUBLISHING, NEW YORK, NY, US, vol. 9, no. 4, 1998, pages 140-145, XP000881929 ISSN: 1043-2760

## Description

### BACKGROUND OF THE INVENTION

In modern society stress and its consequences are prevalent and result in considerable distress, alterations in physical health and social and occupational functioning. At its extreme, stress can lead to disabling neuropsychiatric problems which include depression, anxiety disorders, post-traumatic stress disorder and other illnesses. Recent studies demonstrate the potent effects of stress on the body and brain. For example, chronic and intense stress can result in alterations in the region of the brain that plays an important role in memory. In addition, stress can negatively impact cardiovascular function, immune function and gastrointestinal physiology.

It is estimated that 10% of the population suffers from depression and another 15% from clinically significant anxiety. This high incidence of stress-related problems is reflected by the fact that approximately 50% of visits to primary care doctors are stress and/or psychologically related.

Current treatments for stress and its disorders are highly sought after and include the traditional anti-anxiety drugs like Valium and Xanax. More recently newer antidepressants like Prozac have been used to treat depression, anxiety and other stress related problems. It is estimated that $6 billion was spent last year in the U.S. on drugs like Prozac. However, these treatments still suffer from lack of efficacy in approximately 30% of individuals and in those that do respond only roughly 50% of them will return to normal function. In addition, these treatments have bothersome side-effects (50% have marked sexual dysfunction) which make treatment with these drugs unacceptable for many individuals. Since depression and anxiety are recurrent and chronic disorders it is important that patients are comfortable taking their medication over a long period of time. Overactivity of the corticotropin-releasing factor CRF system is implicated in depression and anxiety and treatments aimed at this system may be very effective.

US 5,965,790 is concerned with SR-BI regulatory sequences, therapeutic methods of use, and agents that modulate SR-BI promoter activity.

### BRIEF SUMMARY OF THE INVENTION

In one embodiment, the present invention is a method for identifying agents that alter the activity of the promoter region of the CRF_{2α} receptor. The method may comprise the steps of (a) obtaining a cell line or organism, wherein the cell line or organism comprises a nucleic acid sequence encoding a promoter sequence of a heterologous CRF_{2α} receptor operably attached to a reporter gene and (b) introducing a test agent into a cell or transgenic animal and evaluating the expression of the reporter gene product compared to a control cell line or transgenic animal wherein the agent has not been introduced into the cell line or transgenic animal.

In preferred embodiments, the CRF_{2α} receptor is a rat receptor or a human receptor.

In another embodiment, the present invention is a transformed cell line, wherein a cell line has been transformed with a construct comprising a nucleic acid sequence encoding a heterologous CRF_{2α} receptor promoter operably connected to a reporter gene or a link to a transgenic animal, wherein the animal has been transfected with a nucleic acid sequence encoding a heterologous CRF_{2α} receptor promoter operably connected to a receptor gene.

It is one object of the present invention to create a transformed cell line or transgenic animal transfected with the nucleic acid sequence encoding a heterologous CRF_{2α} receptor promoter operably connected to a reporter gene.

The invention also provides a method of determining which region of the receptor promoter interacts with a test agent, comprising the steps of
(a) obtaining multiple cell lines or organisms, wherein the cell lines or organisms comprise a construct comprising a nucleic acid sequence encoding a fragment of the promoter sequence of a heterologous CRF_{2α} receptor operably attached to a reporter gene, wherein the multiple cell lines or organisms comprise constructs comprising different fragments,
(b) introducing a test agent into the cells or transgenic animals and evaluating the expression if the reporter gene product compared to a control cell line or transgenic animal wherein the agent has not been introduced into the cell line or transgenic animal,
(c) comparing the expression of the reporter gene from the multiple constructs, and
(d) identifying the constructs comprising fragments which interact with the test agent.

It is another object of the present invention to screen compounds or identify agents that alter the activity of the CRF_{2α} receptor promoter region.

Other objects, advantages and features of the present invention will become apparent after analysis and review of the claims, specification and drawings.

### DETAILED DESCRIPTION OF THE SEVERAL VIEWS OF THE DRAWINGS

Fig. 1 is the representation of rat and human CRF₂ genomic clones.

Fig. 2 is a comparison of the promoter regions for the rat and human CRF_{2α} receptor gene.

Fig. 3 is a bar graph demonstrating basal expression of CRF_{2α} promoter fragments.

### DESCRIPTION OF THE INVENTION

### A. In General

The present invention relates to corticotropin-releasing factor (CRF), which is a hormone and neurotransmitter thought to integrate the various electrophysiological, immune, endocrine and behavioral responses to stress.

Studies in animals demonstrate that antagonism of the CRF system blocks the distress and physical effects related to stress. Studies in humans show that the CRF system in the brain is overactive in patients with depression, anxiety disorders and other neuropsychiatric problems. In addition, human and animal studies demonstrate that many effective antidepressant treatments decrease brain CRF activity. Based on these findings the pharmaceutical industry is currently intensively searching for orally administered compounds that will block or reduce the effects of CRF in the brain. Already some compounds have been identified and are in the early stages of human studies.

The CRF system is now known to consist of five components. CRF is a neurotransmitter that is released from neurons and has its effects by interacting with CRF receptors located on adjacent brain cells. Urocortin is another neurotransmitter similar to CRF that also interacts with the system. Once stimulated the receptors activate intracellular processes which mediate the stress effects.

CRF produces its effects by interacting with two different receptors termed CRF₁ and CRF₂. There also exists at least three different splice variants of the CRF₂ receptor, termed "CRF_{2α}," "CRF_{2β}" and "CRF_{2γ}." In addition to CRF₁ and CRF₂ receptors, there also exists a protein that is found in brain cells that functions to inactivate CRF after it is released termed "CRF binding protein."

While much is known about the biology of CRF, considerably less is understood about CRF₁, CRF₂ and the binding protein. Most believe that CRF₁ receptor is responsible for mediating the effects of stress and also may be important in depression and anxiety. However, other evidence suggests that CRF₂ receptor may also play a critical role in mediating the effects of stress. The pharmaceutical industry has targeted CRF₁ for the development of antagonists to block the effects of stress. While interest in CRF₂ may exist, small molecule antagonists specific for this receptor remain to be discovered.

The present invention invokes a different therapeutic approach aimed at altering the regulation of the gene encoding the CRF₂ receptor and has the potential to be a more effective strategy in the treatment of anxiety, depression and other stress-related problems. This approach is based on the hypothesis that the primary problem in these illnesses is over-expression of CRF and/or its receptors. Thus, a treatment aimed at the primary cause of these problems should prove more effective and be without non-specific effects on other systems. For example, drugs that control the regulation of CRF or its receptors would allow greater precision in stress management. Traditional approaches suffer from numerous unwanted effects because receptor antagonists affect all receptors throughout the brain and body and do not selectively interact with those regions that are most important in an illness.

The advantage of understanding and developing drugs to affect regulation of genes that make receptors and other proteins is that they can be directed to alter levels of proteins in specific tissues. For example, the amygdala is located deep in the brain and is thought to be pivotal in mediating the effects of CRF in depression and anxiety. Once the factors that regulate the selective expression of CRF in the amygdala are identified, drugs could be targeted to affect CRF only in this region, leaving other sites (cortex, brain stem, heart, hypothalamus) unaffected.

For the purposes of the present invention we have cloned and identified the promoter region of the rat CRF₂ receptor gene. This promoter region of the gene is responsible for determining where in the body and when during development the CRF₂ receptor is expressed. This region also controls how much receptor is expressed. Therefore, we envision that the promoter region would be a target for drug development for the treatment of various psychopathologies described above, including depression, generalized anxiety, social anxiety, post traumatic stress and panic disorder. Using the promoter region of the gene in a cell and/or chip based screening assay will allow us to develop methods to identify agents that alter the activity of the promoter region and, thus, affect the expression of the CRF₂ receptor. These agents could have significant therapeutic potential in the treatment of various psychopathologies.

### B. Human CRF₂ receptor gene

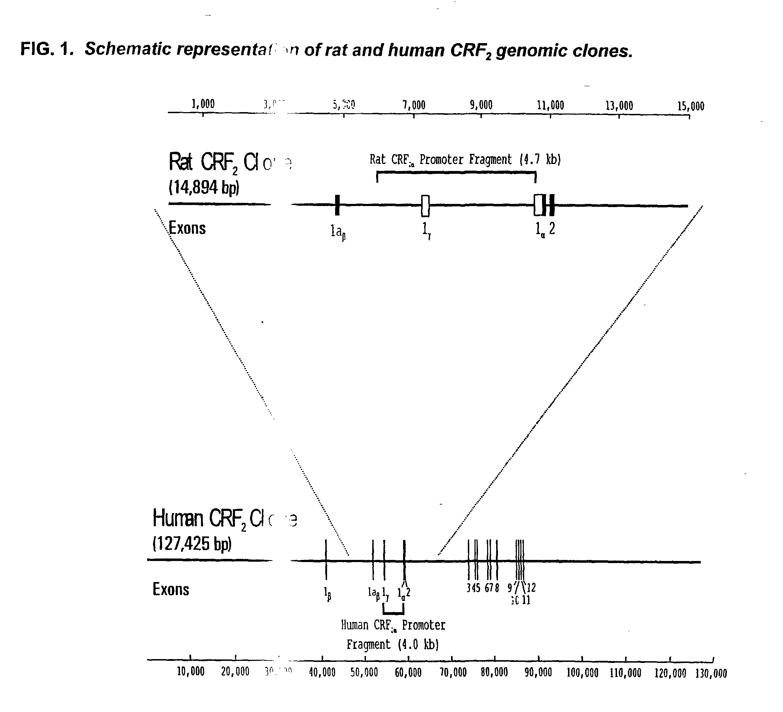

The clone containing the entire gene for the human CRF₂ receptor was obtained from Research Genetics (Huntsville, AL). This PAC clone (RP5-1143H19) contained a 127,425 bp insert, which included the first exons for the CRF_{2α}, CRF_{2β} and CRF_{2γ}receptors and remaining 11 exons that are common to all three isoforms (see Fig. 1). The clone contains approximately 42,000 bp upstream of exon 1 of the CRF_{2α}, and approximately 39,000 bp downstream of the final exon.

### C. Rat CRF₂ receptor gene

The rat CRF₂ receptor gene was cloned from a Sprague-Dawley rat genomic library constructed in Lambda FIX® II obtained from Stratagene (La Jolla, CA). The library was prepared from a partial Sau3A I digest of kidney DNA obtained from male rats (16 months old). The library was probed with a ³²P-labelled fragment of the rat CRF_{2α} cDNA (T.W. Lovenberg, et al., Proc. Natl. Acad. Sci. USA 92(836-840):PNAS57, 1995), which corresponded to bases 1 to 261 of the cDNA (Genbank # U16253). The single positive clone that was obtained was plaque purified, the insert was excised by *Not* I digestion and subcloned into the pGEM-5Zf(+) vector (Promega, Madison, WI). The entire insert was sequenced using the GPS-1 Genome Priming System (New England Biolabs, Beverly, MA) which uses randomly interspersed primer binding sites.

The insert was determined to be 14,894 bp long, and the intron/exon junctions were identified by comparison of the insert sequence to that of rat CRF_{2α} (Genbank # U16253), mouse CRF_{2β} (Genbank # U21729) and human CRF_{2γ} (Genbank # AF019381) cDNAs. This revealed that the clone contained the first exons of the CRF_{2α} and second exon (1a) of the CRF_{2β} (Fig. 1). The clone also contained exon 2, which is common to each of the isoforms. ln addition, the clone contained a region that corresponds to the first exon of the CRF_{2γ}; however, it lacks the necessary consensus splice site sequences and ATG translation start site to function as an exon.

### D. Comparison of rat and human CRF₂ gene sequences

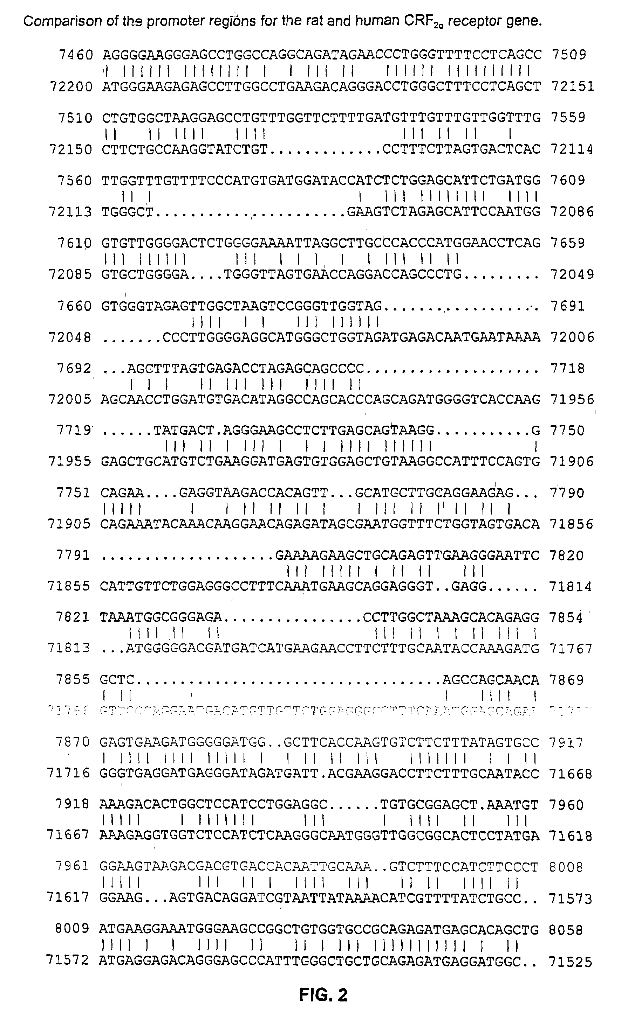

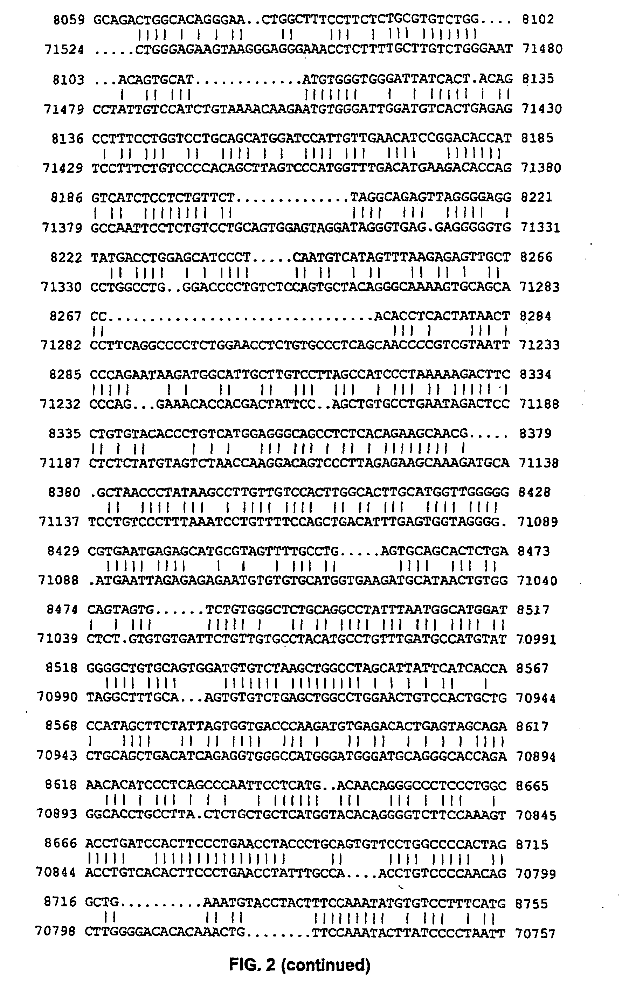

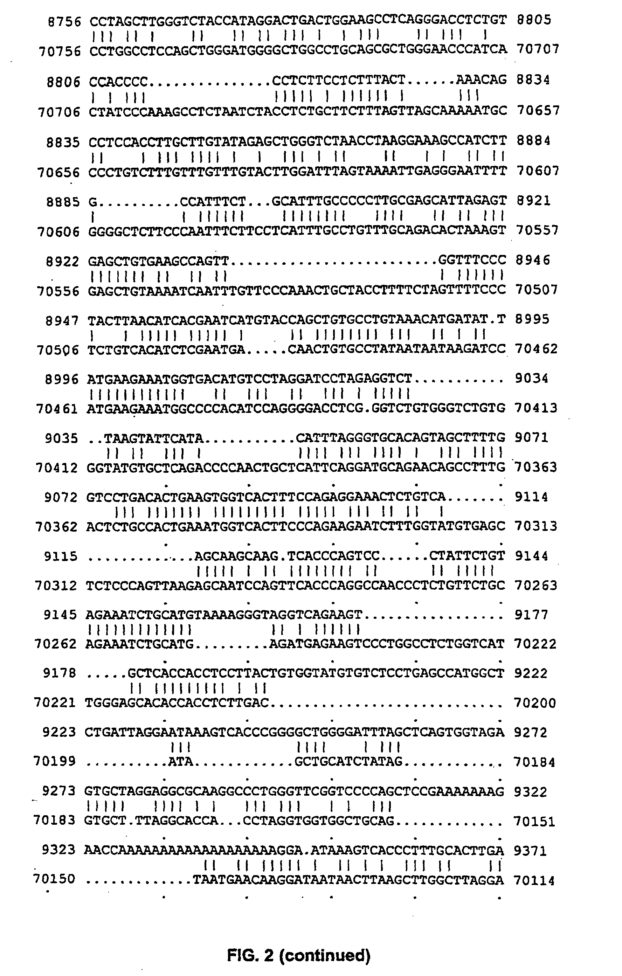

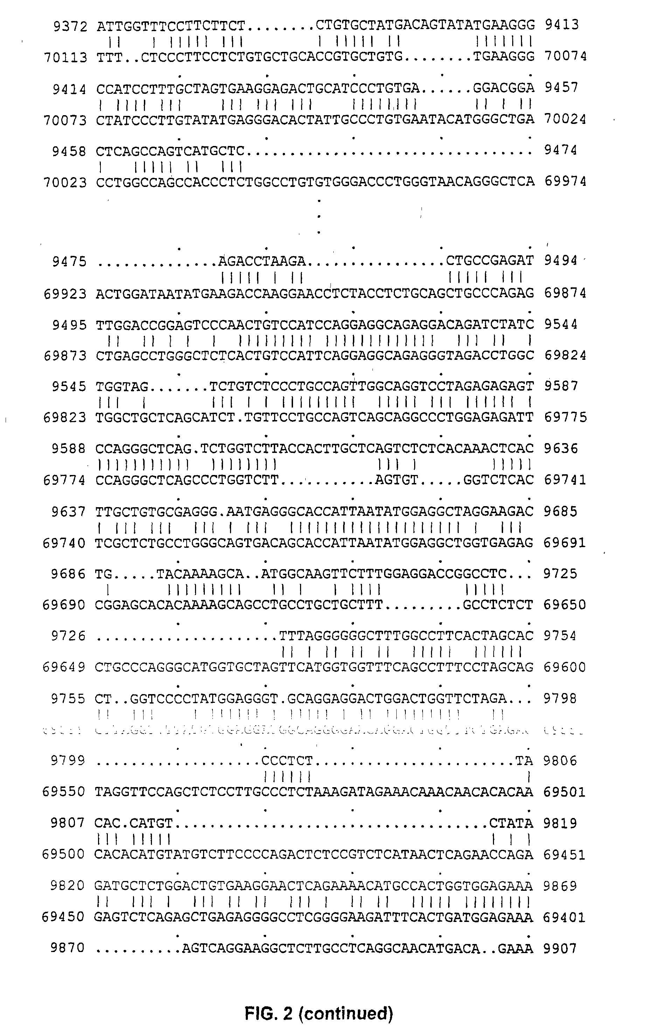

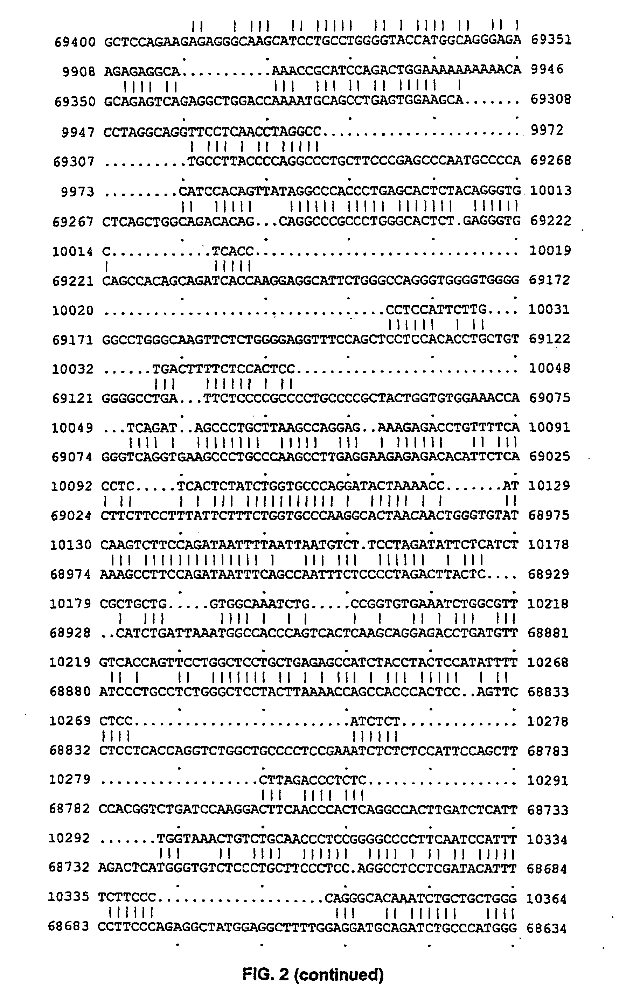

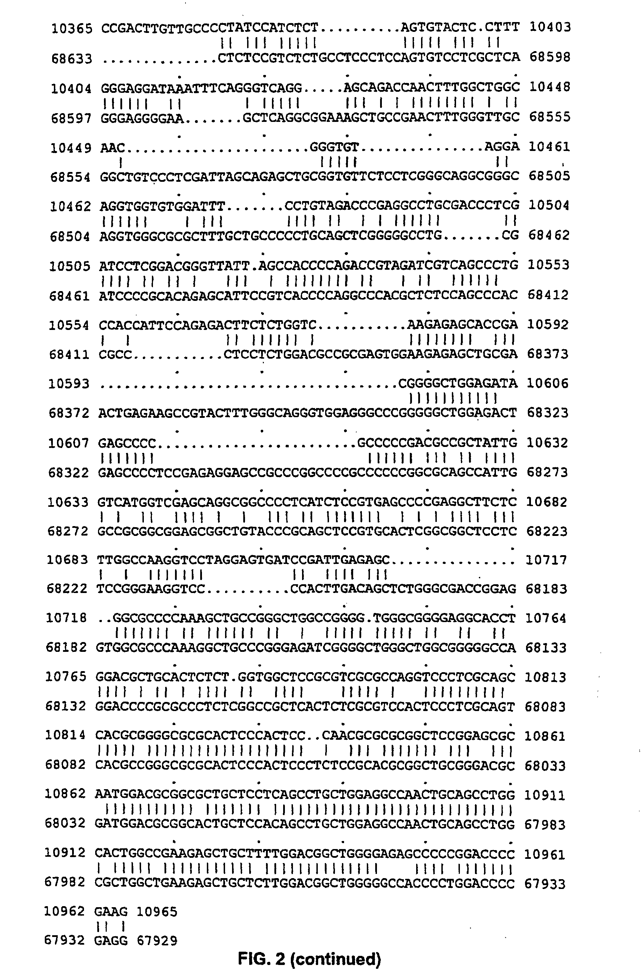

We identified the region of the human CRF₂ gene that corresponds to the rat CRF₂ genomic clone (see Fig. 1). The promoter region for the CRF_{2α} should be located within the ∼4000 bp of sequence that lie upstream of the first exon for the CRF_{2α} but downstream of the first CRF_{2γ} exon. We compared the rat and human CRF₂ gene sequences in a subregion of this fragment that contains the first 2000 bp immediately upstream of the first CRF_{2α} exon using the BestFit program from the Genetics Computer Group (GCG) Wisconsin Package version 10.0. The gap creation penalty was set at 40 and the gap extension penalty was set at 2. The analysis revealed 70.4% identity between the two sequences (see Fig. 2). It is likely that both mouse and monkey sequences will have greater than 70.4% identity compared to rat and human, respectively.

Transcription factor-binding sites are short sequences of DNA located in promoter regions where transcription factors bind to exert their effect on gene regulation. These sites have been found to confer unique expression properties to genes in other systems and are likely important for the temporal and spatial regulation of the CRF₂ receptor gene. They also serve to highlight the basal promoter, which is the region of the CRF₂ receptor promoter that is most critical for appropriate developmental and cell-specific expression of the gene.

To identify potential transcription factor binding sites, analysis was performed on 2000 bp of sequence immediately upstream of the first CRF_{2α} exon start site in both the rat and human sequences using Matlnspector v2.2 (K.K. Quandt, et al., Nucleic Acids Res. 23(23):4878-4884, 1995), public domain software with the Transtac 4.0 vertebrate matrices (T. Heinemeyer, et al., Nucleic Acids Res. 27(1):318-322, 1999). The threshold levels were set at 1.0 for core similarity and 0.9 for matrix similarity. This identified 152 and 146 potential transcription factor binding sites in the human and rat CRF_{2α} promoter regions, respectively.

Numerous potential transcription factor binding sites are present within any given promoter sequence. Very few of these are ultimately functionally relevant. A comparison between the same promoter from two different species allows one to identify those elements that are conserved and therefore likely to be critical for the appropriate functioning of the gene. Comparison of the human and rat results revealed 51 putative binding sites that were conserved in terms of location and orientation within the two sequences. These transcription factor-binding sites are listed in Table 1. The location in the table refers to the position of the sequence within the 2000 bp that are immediately upstream of the transcription start site. Because these sites are conserved between rat and human we feel they may constitute important regulatory elements.

### E. Preparation of CRF_{2α} receptor promoter constructs

The minimal promoter fragment within the human and rat CRF_{2α} receptor genes that confers the correct temporal and spatial expression of the CRF_{2α} receptor will be subcloned into an expression vector that contains either the firefly luciferase (pGL3-basic Promega, Madison, WI) or enhanced green fluorescent protein as a reporter (Clontech, Palo Alto, CA).

### i. Human CRF_{2α} receptor promoter

To obtain the fragment corresponding to the promoter region of the CRF_{2α} gene, it was necessary to first subclone into an intermediate vector, pRL-null (Promega, Madison, WI) prior to subcloning into the reporter construct that will be used to transfect cells. A 4040 bp fragment of the human CRF₂ gene corresponding to the promoter region of the CRF_{2α} receptor (see Fig. 1) was excised with the restriction enzymes *Nar*I and *Nde*I. The fragment was subcloned into the vector pRL-null that had been digested with the same two enzymes. This insert was then removed from the pRL-null construct with *Xho*I and *EcoR*I and subcloned into the pEGFP-1 vector that had been digested with the same two enzymes. We also subcloned this fragment into a luciferase reporter, pGL-3 basic (Promega). The insert was removed from pRLRVI with *EcolcR*I and *Sal*I and inserted into pGL3-basic that had been digested with *Sma*I and *Xho*I.

We focused on the first 2000 bp in our sequence comparison and found a 70.4% identity between the rat and human sequence. Although we will initially examine a fragment containing 4040 bp of sequence, we know that a smaller fragment that has been deleted from the 5' end will function as the basal promoter. Using a common reverse (3') primer that ended 36 bp downstream of the putative transcription start point (TSP), we generated sequentially smaller fragments of the CRF_{2α} promoter region through PCR with several forward (5') primers. The constructs generated were from -3898, -3406, -2883, -2346, -1375, -840; and -346 bp relative to the TSP through +36 bp (referred to as the -3898, -3406, -2883, -2346, -1375, -840, and -346 constructs respectively). Our goal is to define the basal promoter, which in some instances has been found to be shorter than 500 bp.

### ii. Rat CRF_{2α} receptor promoter

A 4693 bp fragment corresponding to the promoter region of the rat CRF_{2α} receptor (see Fig. 1) will be obtained by digestion with *Hind*III and *Bsr*BI. This will be subcloned into the *Hind*III and *Sma*I sites of the pEGFP-1 vector. This fragment will also be subcloned into a luciferase reporter, pGL-3 basic (Promega). To generate smaller fragments of the rat CRF_{2α} promoter, we will use a strategy identical to that described for the human CRF_{2α} promoter.

**Table 1.**

| Location of conserved putative transcription factor binding sites. | | |
|---|---|---|
| | Position (strand) of Binding Site | |
| Binding Site Name | Rat | Human |
| AP1FJ_Q2 | 131 (+) | 230 (+) |
| AP1FJ_Q2 | 427 (+) | 533 (+) |
| AP1FJ_Q2 | 459 (-) | 457 (-) |
| AP1FJ_Q2 | 702 (-) | 908 (-) |
| AP1FJ_Q2 | 1567 (-) | 1347 (-) |
| AP1FJ_Q2 | 1892 (-) | 1812 (-) |
| AP1_Q2 | 437 (-) | 457 (-) |
| AP1_Q2 | 1567 (-), | 1347 (-) |
| AP1_Q2 | 1892 (-) | 1812 (-) |
| AP4_Q5 | 322 (+) | 370 (+) |
| AP4_Q5 | 322 (-) | 370 (-) |
| AP4_Q5 | 1705 (-) | 1721 (-) |
| CREB_02 | 1893 (-) | 1813 (-) |
| DELTAEF1_01 | 15 (+) | 45 (+) |
| DELTAEF1_01 | 189 (+) | 85 (+) |
| DELTAEF1_01 | 1102 (+) | 1124 (+) |
| DELTAEF1_01 | 1812 (-) | 1751 (-) |
| E47 02 | 1100 (-) | 1122 (-) |
| GATA1_02 | 338 (+) | 334 (+) |
| GATA1_02 | 1400 (+) | 1290 (+) |
| GATA1_03 | 1489 (+) | 1290 (+) |
| GATA1_03 | 1728 (-) | 1372 (-) |
| GATA1_04 | 1401 (+) | 1291 (+) |
| GATA1_04 | 1450 (-) | 1372 (-) |
| GATA1_05 | 1491 (+) | 1292 (+) |
| GATA_C | 1493 (+) | 1294 (+) |
| GC_01 | 1960 (-) | 1959 (-) |
| GKLF_01 | 165 (-) | 150 (-) |
| IK2_01 | 27 (-) | 15 (-) |
| IK2_01 | 144 (+) | 34 (+) |
| IK2_01 | 292 (-) | 184 (-) |
| IK2_01 | 791 (-) | 769 (-) |
| IK2_01 | 997 (+) | 898 (+) |
| IK2_01 | 1687 (-) | 1705 (-) |
| LMO2COM_01 | 1102 (-) | 1124 (-) |
| LMO2COM_02 | 1452 (-) | 1374 (-) |
| MYOD_01 | 1102 (-) | 1124 (-) |
| MYOD_Q6 | 1103 (+) | 1125 (+) |
| MZF1_01 | 601 (+) | 680 (+) |
| MZF1_01 | 656 (-) | 773 (-) |
| MZF1_01 | 1112 (-) | 1149 (-) |
| MZF1_01 | 1691 (-) | 1798 (-) |
| NF1_Q6 | 1791 (+) | 1981 (+) |
| NFAT_Q6 | 727 (-) | 645 (-) |
| NFAT_Q6 | 1282 (+) | 1172 (+) |
| NFAT_Q6 | 1824 (-) | 1569 (-) |
| NFY_01 | 1976 (-) | 1976 (-) |
| NFY_Q6 | 1979 (-) | 1979 (-) |
| NKX25_01 | 718 (-) | 1512 (-) |
| S8_01 | 1492 (-) | 1293 (-) |
| SP1_Q6 | 1961 (-) | 1960 (-) |

### F. Production of transfected cell lines

In one embodiment, the present invention is a transfected cell line. One preferred method of creating such a cell line is described as follows: The constructs described above containing the human or rat promoter fragments placed upstream of the firefly luciferase gene will be used to transfect immortalized cell lines. The constructs will be transfected into CHO-K1 and A7R5 cell lines using lipofectamine 2000 (Life Technologies, Rockville, MD). Primary cultures of the central nervous system, as well as additional immortalized cell lines, are also appropriate for these transfections. To control for transfection efficiency, the cells will also be co-transfected with the pRL-TK vector (Promega, Madison, WI). The pRL-TK vector contains the *Renilla* luciferase gene downstream of the herpes simplex virus thymidine kinase promoter, a promoter which provides low to moderate levels of expression. Cell lysates will be assayed for total protein using the BCA assay (Pierce, Rockford, IL) to standardize for the protein extraction. The level of reporter gene expression from a standardized amount of cell extract will be quantified by measuring luciferase activity using a luminometer (EG&G Wallac, Gaithersburg, MD) and the dual-luciferase reporter assay system (Promega, Madison, WI). Firefly luciferase activity will reflect CRF_{2α} receptor promoter activity and *Renilla* luciferase activity will be used to normalize data between experiments.

### G. Characterization of basal expression from CRF_{2α} receptor promoter fragments

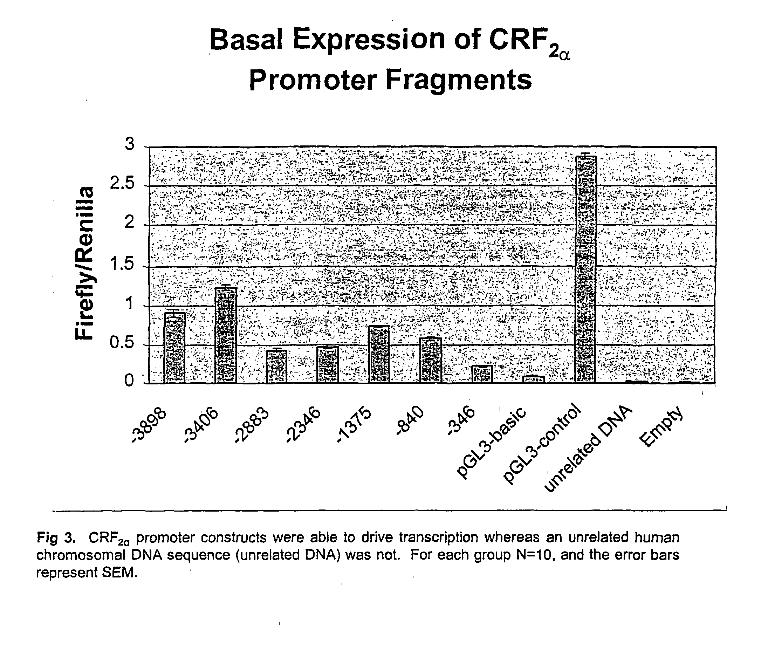

Using the methods described above, transient transfections of CHO-K1 cultures were assayed for reporter gene expression (See Fig. 3). In these experiments, four basic controls were utilized. The cultures referred to as empty were not transfected with any construct. The empty cultures served to demonstrate background luminescence of the CHO-K1 cultures. The cultures referred to as pGL-3 basic were transfected with a pGL-3 firefly luciferase reporter construct that did not contain an experimental promoter, and with the pRL-TK vector. These cultures should demonstrate a very low level of expression and may be considered a negative control. The cultures referred to as pGL-3 control were transfected with a construct containing the firefly luciferase reporter downstream of the SV40 viral promoter as well as the pRL-TK vector. These cultures should demonstrate a very high level of expression and may be considered a positive control. Finally, the cultures referred to as unrelated DNA were transfected with a construct containing 1916 bp of DNA sequence upstream of the firefly reporter gene and with the pRL-TK vector. The 1916 bp of this construct were a random DNA sequence with the final 21 bp most 3' being identical to our putative promoter constructs. These cultures were intended to demonstrate the specificity of our promoter constructs.

Our results indicate that the -3406 construct has the highest level of expression of the CRF_{2α} promoter constructs (See Fig. 3). Levels of expression generally decrease as the promoter's length is shortened, reaching a minimum with the -346 construct. 1-way ANOVA revealed a significant difference amongst constructs [F(10,83)=864, P<0.001]. Post-hoc analysis with Bonferroni/Dunn adjustment of a value indicated that the -3406 construct was significantly different from both the pGL3-basic and pGL 3 control constructs (P < .001 for both), and the -346 construct was also significantly different from pGL3-basic and pGL 3 control constructs (P < .001 for both). Examination of the means indicate that our lowest levels of expression (-346 construct) appear to be 141% greater than housekeeping levels of expression (pGL-3 basic) (mean -346 = 0.222 ± .008, mean pGL3 basic = .092 ± .004), and they are 8% of the strong expression elicited by the viral promoter (pGL-3 control) (mean -346 = 0.222 ± .008, mean pGL3 control = 2.878 ± .041). Our highest levels of expression (-3406 construct) appear to be 1242% greater than housekeeping levels of expression (pGL-3 basic) (mean -3406 = 1.229 ± .042, mean pGL3 basic = .092 ± .0.004, and they are 43% of the strong expression from the SV40 promoter (pGL-3 control) (mean -3406 = 1.229 ± 0.42, mean pGL3 control = 2.878 ± 0.41). Furthermore, an unrelated human chromosomal DNA sequence was not able to drive expression above background. Since all the CRF_{2α} promoter constructs function effectively, they may be utilized to isolate the promoter sequence responsible for a given candidate drug effect. By comparing the expression in a construct with and without a given candidate drug to the expression in the other constructs with and without the treatment, the precise region of the promoter associated with a given candidate drug's effect may be determined. Thus, it appears that the CRF_{2α} promoter constructs function and will be appropriate tools to monitor CRF_{2α} specific transcription.

### H. Production of transgenic mice

In another embodiment, the present invention is a transgenic mouse comprising a heterologous promotor sequence for corticotropin releasing hormone receptors CRF_{2α}. In one preferred embodiment, the transgenic mouse would be created as follows: Once potential therapeutic agents are identified in our cell culture model we will test their ability to alter CRF₂ receptor promoter activity in transgenic animals. Reporter constructs that consist of the basal CRF_{2α} receptor promoter placed upstream of the enhanced green fluorescent protein or β-galactosidase will be used to generate transgenic mice. The procedure for generating the enhanced green flourescent construct has already been described, and the procedure for generating the β-galactosidase construct was identical to that used to make the firefly luciferase construct. These animals will allow us to confirm the appropriate spatial and temporal expression of the CRF_{2α} receptor promoter.

The reporter constructs will be identical to those described above and will preferably consist of 4040 bp of human CRF_{2α} receptor promoter or 4693 bp of rat CRF_{2α} receptor promoter fused to the coding region of EGFP or $-galactosidase. Transgenic animals will be generated using standard techniques. The preferred technique would involve the microinjection of 100 copies of the promoter-reporter construct into the male pronucleus of a fertilized egg. Injected eggs are then transplanted into pseudo-pregnant females and the progeny from these transplantations examined for the presence of the CRF_{2α} receptor promoter-reporter construct (called "the transgene"). Animals containing the transgene will be identified by extracting DNA from a small amount of tail tissue and probing this DNA with a segment of the EGFP or β-galactosidase gene, which is not normally found in the mammalian genome. Animals that contain the CRF_{2α} receptor promoter-reporter transgene will be mated to normal animals so that transgenic lines are established. Preferably, we will generate three transgenic lines that contain the transgene in three separate sites within the genome. In this way we will verify that the expression patterns we observe are a result of EGPF or β-galactosidase expression from our promoter segment and are not due to site insertion effects.

To confirm the appropriate function and expression of the CRF_{2α} receptor promoter-reporter transgene, the following will preferably be performed: Brain tissue sections will be taken from transgenic animals beginning in late embryonic development and extending at five-day intervals into adulthood (postnatal day 60). Sections will then be observed under 488 nm light or 420 nm light to identify those brain cells that express EGFP or β-galactosidase, respectively. The pattern of reporter expression will be compared with the normal pattern of CRF_{2α} receptor expression. The expression of the CRF_{2α} receptor promoter transgene should overlap with expression of the endogenous CRF_{2α} receptor gene both temporally (i.e., it should begin to expressed when CRF_{2α} receptor is first expressed) and spatially (i.e., expression of the transgene should be confined to those cells within septum and ventromedial hypothalamus that normally express CRF_{2α} receptor).

### I. Use of Transformed Cell Lines and Transgenic Animals

Cells transfected with CRF_{2α} receptor promoter regions fused to a reporter construct will allow the testing of potential therapeutics. Pharmacologically relevant amounts of candidate small molecules will be applied to the transfected cells in the media and the influence of these molecules on reporter gene expression levels will be assessed by the methods discussed above. These experiments will be replicated at least 10 times and any small molecule that yields a statistically significant difference in expression will be considered a positive find. The level of reporter expression after treatment with a specific candidate drug will enable the determination of the degree to which the drug is influencing CRF_{2α} receptor activity.

Candidates that increase the expression of CRF₂ promoter-reporter activity can then be further tested *in vivo*. Transgenic animals will be treated with the candidate drug to determine whether CRF_{2α} promoter-reporter transgene levels are elevated in the same way and to the same degree as that found in the cells lines. Adverse drug effects can also be determined with these animals.

If the drug behaves similarly *in vivo* and there are no signs of significant toxicity, then the drug could be tested in a variety of animal models that are predictive of antidepressant or anti-anxiety activity. If the candidates are active in these tests they could serve as therapeutic agents in psychiatric disorders, such as depression.

## Claims

1. A method for identifying agents that alter the activity of the promoter region of the CRF_{2α} receptor, comprising the steps of
(a) obtaining a cell line or organism, wherein the cell line or organism comprises a nucleic acid sequence encoding a promoter sequence of a heterologous CRF_{2α} receptor operably attached to a reporter gene, and
(b) introducing a test agent into the cell or organism and evaluating the expression of the reporter gene product compared to a control cell line or organism wherein the agent has not been introduced into the cell line or organism.

2. The method of claim 1 wherein the CRF_{2α} receptor is a rat receptor.

3. The method of claim 1 wherein the CRF_{2α} receptor is the human receptor.

4. The method of claim 2 wherein the CRF_{2α} receptor promoter region comprises the 4693 bp upstream of the transcription start site for the rat CRF_{2α} receptor.

5. The method of claim 2 wherein the promoter region comprises a deletion fragment of the rat CRF_{2α} receptor-4693 bp upstream promoter, wherein the fragment retains at least 50% of the ability to drive transcription as compared to the -4693 bp upstream promoter.

6. The method of claim 3 wherein the CRF_{2α} receptor promoter region comprises the 4031 bp upstream of the transcription start site for the human CRF_{2α} receptor.

7. The method of claim 3 wherein the CRF_{2α} receptor promoter region comprises at least 3898 bp upstream of the transcription start site for the human CRF_{2α} receptor.

8. The method of claim 3 wherein the CRF_{2α} receptor promoter region comprises at least 3406 bp upstream of the transcription start site for the human CRF_{2α} receptor.

9. The method of claim 3 wherein the CRF_{2α} receptor promoter region comprises at least 1375 bp upstream of the transcription start site for the human CRF_{2α} receptor.

10. The method of claim 3 wherein the CRF_{2α} receptor promoter region comprises at least 840 bp upstream of the transcription start site for the human CRF_{2α} receptor.

11. The method of claim 3 wherein the CRF_{2α} receptor promoter region comprises at least 346 bp upstream of the transcription start site for the human CRF_{2α} receptor.

12. The method of claim 3 wherein the CRF_{2α}, receptor promoter region comprises at least 2346 bp upstream of the transcription start site for the human CRF_{2α} receptor.

13. The method of claim 3 wherein the CRF_{2α} receptor promoter region comprises at least 2883 bp upstream of the transcription start site for the human CRF_{2α} receptor.

14. A transformed cell line, wherein a cell line has been transformed with a construct comprising a nucleic acid sequence encoding a heterologous CRF_{2α} receptor promoter operably connected to a reporter gene.

15. The transformed cell line of claim 14, wherein the cell line is transformed with a construct comprising a rat CRF_{2α} receptor promoter.

16. The transformed cell line of claim 14, wherein the cell line is transformed with a construct comprising human CRF_{2α} receptor promoter.

17. The cell line of claim 16, wherein the CRF_{2α} receptor promoter comprises at least 3406 bp upstream of the transcription start site for the human CRF_{2α} receptor.

18. The cell line of claim 16, wherein the CRF_{2α} receptor promoter comprises at least 346 bp upstream of the transcription start site for the human CRF_{2α} receptor.

19. A transgenic animal, wherein the animal has been transfected with a nucleic acid sequence encoding a heterologous CRF_{2α} receptor promoter operably connected to a reporter gene.

20. A method of determining which region of the CRF_{2α} receptor promoter interacts with a test agent, comprising the steps of
(a) obtaining multiple cell lines or organisms, wherein the cell lines or organisms comprise a construct comprising a nucleic acid sequence encoding a fragment of the promoter sequence of a heterologous CRF_{2α} receptor operably attached to a reporter gene, wherein the multiple cell lines or organisms comprise constructs comprising different fragments,
(b) introducing a test agent into the cells or transgenic animals and evaluating the expression of the reporter gene product compared to a control cell line or transgenic animal wherein the agent has not been introduced into the cell line or transgenic animal,
(c) comparing the expression of the reporter gene from the multiple constructs, and
(d) identifying the constructs comprising fragments which interact with the test agent.

## Patentansprüche

1. Verfahren zum Identifizieren von Mitteln, die die Aktivität der Promotorregion des CRF_{2α}-Rezeptors verändern, umfassend die folgenden Stufen:
(a) Gewinnen einer Zelllinie oder eines Organismus, wobei die Zelllinie oder der Organismus eine Nucleinsäuresequenz, die für eine Promotorsequenz eines heterologen CRF_{2α}-Rezeptors kodiert, in funktioneller Verknüpfung mit einem Reportergen umfasst, und
(b) Einführen eines Testmittels in die Zelle oder den Organismus und Bewerten der Expression des Reportergenprodukts im Vergleich zu einer Kontrollzelllinie oder einem Kontrollorganismus, bei denen das Mittel nicht in die Zelllinie oder den Organismus eingeführt worden ist.

2. Verfahren nach Anspruch 1, wobei es sich beim CRF_{2α}-Rezeptor um einen Rattenrezeptor handelt.

3. Verfahren nach Anspruch 1, wobei es sich beim CRF_{2α}-Rezeptor um den humanen Rezeptor handelt.

4. Verfahren nach Anspruch 2, wobei die CRF_{2α}-Rezeptorpromotorregion die 4693 Basenpaare (bp) strangaufwärts von der Transkriptionsstartzelle für den CRF_{2α}-Rezeptor umfasst.

5. Verfahren nach Anspruch 2, wobei die Promotorregion ein Deletionsfragment des Ratten-CRF_{2α}-Rezeptor-(-4693 bp)-Strangaufwärtspromotors umfasst, wobei das Fragment mindestens 50 % der Fähigkeit zur Steuerung der Transkription im Vergleich zum 4693 bp-Strangaufwärtspromotor behält.

6. Verfahren nach Anspruch 3, wobei die CRF_{2α}-Rezeptorpromotorregion die 4031 bp strangaufwärts von der Transkriptionsstartstelle für den humanen CRF_{2α}-Rezeptor umfasst.

7. Verfahren nach Anspruch 3, wobei die CRF_{2α}-Rezeptorpromotorregion mindestens 3898 bp strangaufwärts von der Transkriptionsstartstelle für den humanen CRF_{2α}-Rezeptor umfasst.

8. Verfahren nach Anspruch 3, wobei die CRF_{2α}-Rezeptorpromotorregion mindestens 3406 bp strangaufwärts von der Transkriptionsstartstelle für den humanen CRF_{2α}-Rezeptor umfasst.

9. Verfahren nach Anspruch 3, wobei die CRF_{2α}-Rezeptorpromotorregion mindestens 1375 bp strangaufwärts von der Transkriptionsstartstelle für den humanen CRF_{2α}-Rezeptor umfasst.

10. Verfahren nach Anspruch 3, wobei die CRF_{2α}-Rezeptorpromotorregion mindestens 840 bp strangaufwärts von der Transkriptionsstartstelle für den humanen CRF_{2α}-Rezeptor umfasst.

11. Verfahren nach Anspruch 3, wobei die CRF_{2α}-Rezeptorpromotorregion mindestens 346 bp strangaufwärts von der Transkriptionsstartstelle für den humanen CRF_{2α}-Rezeptor umfasst.

12. Verfahren nach Anspruch 3, wobei die CRF_{2α}-Rezeptorpromotorregion mindestens 2346 bp strangaufwärts von der Transkriptionsstartstelle für den humanen CRF_{2α}-Rezeptor umfasst.

13. Verfahren nach Anspruch 3, wobei die CRF_{2α}-Rezeptorpromotorregion mindestens 2883 bp strangaufwärts von der Transkriptionsstartstelle für den humanen CRF_{2α}-Rezeptor umfasst.

14. Transformierte Zelllinie, wobei eine Zelllinie mit einem Konstrukt transformiert worden ist, das eine Nucleinsäuresequenz, die für einen heterologen CRF_{2α}-Rezeptorpromotor kodiert, in funktioneller Verknüpfung mit einem Reportergen umfasst.

15. Transformierte Zelllinie nach Anspruch 14, wobei die Zelllinie mit einem Konstrukt, das einen Ratten-CRF_{2α}-Rezeptorpromotor umfasst, transformiert worden ist.

16. Transformierte Zelllinie nach Anspruch 14, wobei die Zelllinie mit einem Konstrukt, das humanen CRF_{2α}-Rezeptorpromotor umfasst, transformiert worden ist.

17. Zelllinie nach Anspruch 16, wobei der CRF_{2α}-Rezeptorpromotor mindestens 3406 bp strangaufwärts von der Transkriptionsstartstelle für den humanen CRF_{2α}-Rezeptor umfasst.

18. Zelllinie nach Anspruch 16, wobei der CRF_{2α}-Rezeptorpromotor mindestens 346 bp strangaufwärts von der Transkriptionsstartstelle für den humanen CRF_{2α}-Rezeptor umfasst.

19. Transgenes Tier, wobei das Tier mit einer Nucleinsäuresequenz, die für einen heterologen CRF_{2α}-Rezeptorpromotor kodiert, in funktioneller Verknüpfung mit einem Reportergen transfiziert worden ist.

20. Verfahren zur Bestimmung der Region des CRF_{2α}-Rezeptorpromotors, die mit einem Testmittel in Wechselwirkung tritt, umfassend die folgenden Stufen:
(a) Gewinnen von mehrfachen Zelllinien oder Organismen, wobei die Zelllinien oder Organismen ein Konstrukt umfassen, das eine Nucleinsäuresequenz, die für ein Fragment der Promotorsequenz eines heterologen CRF_{2α}-Rezeptors kodiert, in funktioneller Verknüpfung mit einem Reportergen umfasst, wobei die mehrfachen Zelllinien oder Organismen Konstrukte umfassen, die verschiedene Fragmente umfassen,
(b) Einführen eines Testmittels in die Zellen oder transgenen Tiere und Bewerten der Expression des Reportergenprodukts im Vergleich mit einer Kontrollzelllinie oder einem transgenen Kontrolltier, wobei das Mittel nicht in die Zelllinie oder das transgene Tier eingeführt worden ist,
(c) Vergleichen der Expression des Reportergens aus den mehrfachen Konstrukten und
(d) Identifizieren der Konstrukte, die Fragmente umfassen, die mit dem Testmittel in Wechselwirkung treten.

## Revendications

1. Procédé permettant d'identifier des agents qui affectent l'activité de la région promoteur du récepteur CRF_{2α}, lequel procédé comporte les étapes suivantes :
a) obtenir une lignée cellulaire ou un organisme qui contienne une séquence d'acide nucléique codant une séquence promoteur d'un récepteur CRF_{2α} hétérologue, opérationnellement attachée à un gène rapporteur,
b) et introduire un agent testé dans la lignée cellulaire ou l'organisme et évaluer l'expression du produit du gène rapporteur, en comparaison avec une lignée cellulaire ou un organisme témoin dans laquelle ou lequel on n'a pas introduit l'agent testé.

2. Procédé conforme à la revendication 1, dans lequel le récepteur CRF_{2α} est un récepteur de rat.

3. Procédé conforme à la revendication 1, dans lequel le récepteur CRF_{2α} est le récepteur humain.

4. Procédé conforme à la revendication 2, dans lequel la région promoteur du récepteur CRF_{2α} comprend les 4693 paires de bases situées en amont du site de démarrage de transcription du récepteur CRF_{2α} de rat.

5. Procédé conforme à la revendication 2, dans lequel la région promoteur comprend un fragment de délétion du promoteur amont de 4693 paires de bases du récepteur CRF_{2α} de rat, lequel fragment conserve, par rapport au promoteur amont de 4693 paires de bases, au moins 50 % de la capacité à induire la transcription.

6. Procédé conforme à la revendication 3, dans lequel la région promoteur du récepteur CRF_{2α} comprend les 4031 paires de bases situées en amont du site de démarrage de transcription du récepteur CRF_{2α} humain.

7. Procédé conforme à la revendication 3, dans lequel la région promoteur du récepteur CRF_{2α} comprend au moins 3898 paires de bases situées en amont du site de démarrage de transcription du récepteur CRF_{2α} humain.

8. Procédé conforme à la revendication 3, dans lequel la région promoteur du récepteur CRF_{2α} comprend au moins 3406 paires de bases situées en amont du site de démarrage de transcription du récepteur CRF_{2α} humain:

9. Procédé conforme à la revendication 3, dans lequel la région promoteur du récepteur CRF_{2α} comprend au moins 1375 paires de bases situées en amont du site de démarrage de transcription du récepteur CRF_{2α} humain.

10. Procédé conforme à la revendication 3, dans lequel la région promoteur du récepteur CRF_{2α} comprend au moins 840 paires de bases situées en amont du site de démarrage de transcription du récepteur CRF_{2α} humain.

11. Procédé conforme à la revendication 3, dans lequel la région promoteur du récepteur CRF_{2α} comprend au moins 346 paires de bases situées en amont du site de démarrage de transcription du récepteur CRF_{2α} humain.

12. Procédé conforme à la revendication 3, dans lequel la région promoteur du récepteur CRF_{2α} comprend au moins 2346 paires de bases situées en amont du site de démarrage de transcription du récepteur CRF_{2α} humain.

13. Procédé conforme à la revendication 3, dans lequel la région promoteur du récepteur CRF_{2α} comprend au moins 2883 paires de bases situées en amont du site de démarrage de transcription du récepteur CRF_{2α} humain.

14. Lignée cellulaire transformée, laquelle lignée cellulaire a été transformée à l'aide d'une construction comprenant une séquence d'acide nucléique codant un promoteur d'un récepteur CRF_{2α} hétérologue, opérationnellement attachée à un gène rapporteur.

15. Lignée cellulaire transformée, conforme à la revendication 14, laquelle lignée cellulaire a été transformée à l'aide d'une construction comprenant un promoteur du récepteur CRF_{2α} de rat.

16. Lignée cellulaire transformée, conforme à la revendication 14, laquelle lignée cellulaire a été transformée à l'aide d'une construction comprenant un promoteur du récepteur CRF_{2α} humain.

17. Lignée cellulaire, conforme à la revendication 16, dans laquelle le promoteur du récepteur CRF_{2α} comprend au moins 3406 paires de bases situées en amont du site de démarrage de transcription du récepteur CRF_{2α} humain.

18. Lignée cellulaire, conforme à la revendication 16, dans laquelle le promoteur du récepteur CRF_{2α} comprend au moins 346 paires de bases situées en amont du site de démarrage de transcription du récepteur CRF_{2α} humain.

19. Animal transgénique, lequel animal a été transfecté à l'aide d'une séquence d'acide nucléique codant un promoteur d'un récepteur CRF_{2α} hétérologue, opérationnellement attachée à un gène rapporteur.

20. Procédé permettant de déterminer quelle région d'un promoteur de récepteur CRF_{2α} interagit avec un agent testé, lequel procédé comporte les étapes suivantes :
a) obtenir plusieurs lignées cellulaires ou plusieurs organismes qui contiennent chacun une construction comprenant une séquence d'acide nucléique codant un fragment d'une séquence promoteur d'un récepteur CRF_{2α} hétérologue, opérationnellement attaché à un gène rapporteur, ces lignées cellulaires ou organismes contenant des constructions qui comprennent des fragments différents,
b) introduire un agent testé dans les lignées cellulaires ou les animaux transgéniques et évaluer l'expression du produit du gène rapporteur, en comparaison avec une lignée cellulaire ou un animal transgénique témoin dans laquelle ou lequel on n'a pas introduit l'agent testé,
c) comparer l'expression du gène rapporteur chez les diverses constructions,
d) et identifier les constructions comprenant les fragments qui interagissent avec l'agent testé.
